# EUROPÄISCHE PATENTANMELDUNG

(11) **EP 0 720 987 A1**
(43) Veröffentlichungstag der Anmeldung: **10.07.1996**
(21) Anmeldenummer: 95120369.4
(22) Anmeldetag: 22.12.1995
(51) Int. Cl.: C07K 5/02, C07D 209/02, A61K 38/55, A61K 31/395

(54) **Neue acylierte Pseudopeptide mit trifluoromethylsubstituiertem 2-Azabicyclooctan**

(30) Priorität: 04.01.1995 DE 19500120
(71) Anmelder: BAYER AG, 51368 Leverkusen (DE)
(72) Erfinder: Raddatz, Siegfried, Dr., D-51065 Köln (DE); Wild, Hanno, Dr., Orange, Connecticut 06477 (US); Häbich, Dieter, Dr., D-42115 Wuppertal (DE); Röben, Wolfgang, Dr., D-51467 Bergisch Gladbach (DE); Hansen, Jutta, Dr., 42115 Wuppertal (DE); Paessens, Arnold, Dr., 42781 Haan (DE); Henninger, Kerstin, Dr., 42115 Wuppertal (DE)

(57) **Zusammenfassung**

Die vorliegende Erfindung betrifft neue acylierte Pseudopeptide mit trifluoromethyl-substituiertem 2-Azabicyclooctan der allgemeinen Formel (I)

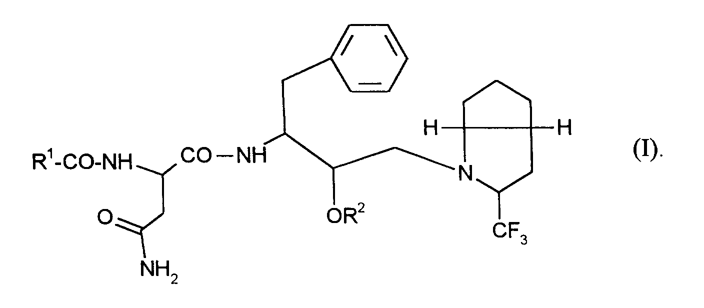

Ein Verfahren zu ihrer Herstellung und ihre Verwendung als retrovirale Mittel.

## Beschreibung

Die vorliegende Erfindung betrifft neue acylierte Pseudopeptide mit trifluoromethyl-substituiertem 2-Azabicyclooctan, ein Verfahren zu ihrer Herstellung und ihre Verwendung als retrovirale Mittel.

Aus der Publikation EP 594 540 A1 sind antiretrovirale Acyl-Verbindungen bekannt.

Die vorliegende Erfindung betrifft neue acylierte Pseudopeptide mit trifluoromethyl-substituiertem 2-Azabicyclooctan der allgemeinen Formel (I) 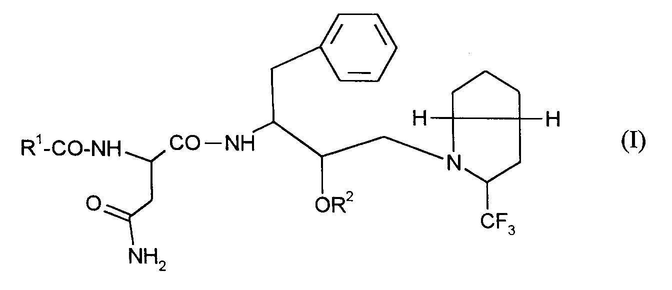
in welcher
- R¹: für einen Rest der Formel 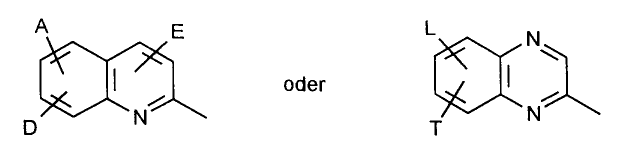 steht,
worin
- A, D, E, L und T: gleich oder verschieden sind und Wasserstoff, Halogen, geradkettiges oder verzweigtes Alkyl mit bis zu 4 Kohlenstoffatomen, Morpholinyl, Phenylthio oder Cycloalkyl mit 3 bis 6 Kohlenstoffatomen bedeuten,
- R²: für geradkettiges oder verzweigtes Acyl mit bis zu 20 Kohlenstoffatomen steht, das gegebenenfalls durch Carboxy, durch geradkettiges oder verzweigtes Alkoxycarbonyl mit bis zu 6 Kohlenstoffatomen oder durch eine Gruppe der Formel -NR³R⁴ substituiert ist,
worin
- R³ und R⁴: gleich oder verschieden sind und Wasserstoff, Phenyl oder geradkettiges oder verzweigtes Alkyl mit bis zu 6 Kohlenstoffatomen bedeuten,
für einen Rest der Formel -CO-NR⁵R⁶ oder P(O)(OR⁷)(OR⁸) steht,
worin
- R⁵ und R⁶: die oben angegebene Bedeutung von R³ und R⁴ haben und mit dieser gleich oder verschieden sind,
- R⁷ und R⁸: gleich oder verschieden sind und Wasserstoff oder geradkettiges oder verzweigtes Alkyl mit bis zu 4 Kohlenstoffatomen bedeuten,
oder für geradkettiges oder verzweigtes Alkoxy mit bis zu 6 Kohlenstoffatomen steht, das gegebenenfalls durch Difluormethyl, Difluormethoxy, Trifluormethoxy oder Trifluormethyl substituiert ist,
oder für einen Rest der Formel 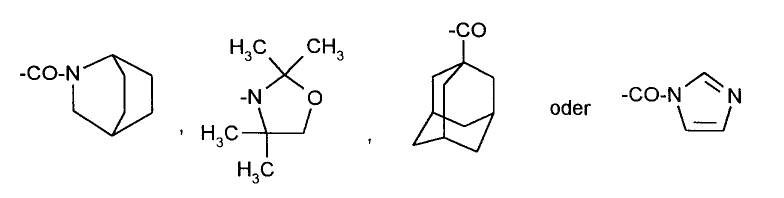 steht,
und deren Salze.

Die erfindungsgemäßen Verbindungen der allgemeinen Formel (I) haben mehrere asymmetrische Kohlenstoffatome.

Der Rest der allgemeinen Formel (A) 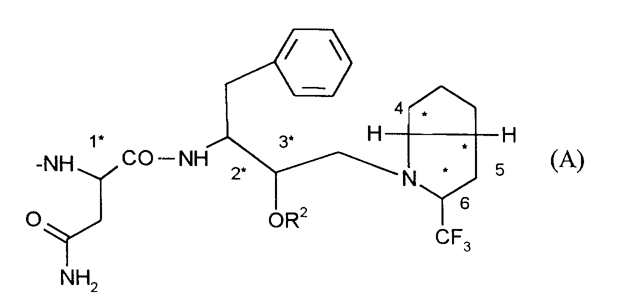
besitzt 6 asymmetrische Kohlenstoffatome (*), die unabhängig voneinander in der R- oder S-Konfiguration vorliegen. Bevorzugt sind die Konfigurationen 1(R), 2(R), 3(S), 4(S), 5(S) und 6(S).

Physiologisch unbedenkliche Salze der acylierten Pseudopeptide mit trifluoromethyl-substituiertem 2-Azabicyclooctan können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Procain, Dibenzylamin, N-Methylmorpholin, Dihydroabiethylamin, 1-Ephedrinamin oder Methyl-piperidin.

Bevorzugt sind Verbindungen der allgemeinen Formel (I),
in welcher
- R¹: für einen Rest der Formel 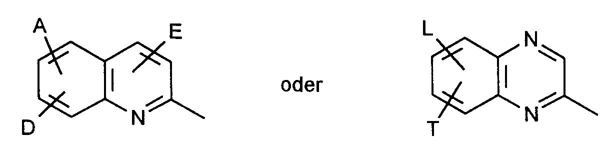 steht,
worin
- A, D, E, L und T: gleich oder verschieden sind und sind und Wasserstoff. Fluor, Chlor, Brom, geradkettiges oder verzweigtes Alkyl mit bis zu 3 Kohlenstoffatomen, Morpholinyl, Phenylthio, Cyclopentyl oder Cylcohexyl bedeuten,
- R²: für geradkettiges oder verzweigtes Acyl mit bis zu 17 Kohlenstoffatomen steht, das gegebenenfalls durch Carboxy, geradkettiges oder verzweigtes Alkoxycarbonyl mit bis zu 4 Kohlenstoffatomen oder durch eine Gruppe der Formel -NR³R⁴ substituiert ist,
worin
- R³ und R⁴: gleich oder verschieden sind und Wasserstoff, Phenyl oder geradkettiges oder verzweigtes Alkyl mit bis zu 4 Kohlenstoffatomen bedeuten,
für einen Rest der Formel -CO-NR⁵R⁶ oder P(O)(OR⁷)(OR⁸) steht,
worin
- R⁵ und R⁶: die oben angegebene Bedeutung von R³ und R⁴ haben und mit dieser gleich oder verschieden sind,
- R⁷ und R⁸: gleich oder verschieden sind und Wasserstoff oder geradkettiges oder verzweigtes Alkyl mit bis zu 3 Kohlenstoffatomen bedeuten,
oder für geradkettiges oder verzweigtes Alkoxy mit bis zu 4 Kohlenstoffatomen steht, das gegebenenfalls durch Difluormethyl, Difluormethoxy, Trifluormethoxy oder Trifluormethyl substituiert ist,
oder für einen heterocyclischen Rest der Formel 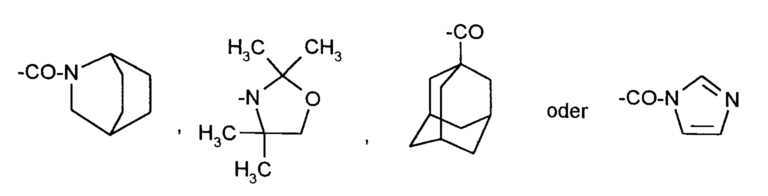 steht,
und deren Salze.

Besonders bevorzugt sind Verbindungen der allgemeinen Formel (I),
in welcher
- R¹: für einen Rest der Formel 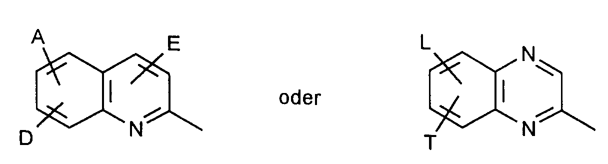 steht,
worin
- A, D, E, L und T: gleich oder verschieden sind und sind und Wasserstoff, Fluor, Chlor, Brom, Methyl, Phenylthio, Cyclopentyl oder Cyclohexyl bedeuten,
- R²: für geradkettiges oder verzweigtes Acyl mit bis zu 15 Kohlenstoffatomen steht, das gegebenenfalls durch Carboxy, geradkettiges oder verzweigtes Alkoxycarbonyl mit bis zu 6 Kohlenstoffatomen oder durch eine Gruppe der Formel -NR³R⁴ substituiert ist,
worin
- R³ und R⁴: gleich oder verschieden sind und Wasserstoff, Phenyl oder geradkettiges oder verzweigtes Alkyl mit bis zu 3 Kohlenstoffatomen bedeuten,
für einen Rest der Formel -CO-NR⁵R⁶ oder P(O)(OR⁷)(OR⁸) steht,
worin
- R⁵ und R⁶: die oben angegebene Bedeutung von R³ und R⁴ haben und mit dieser gleich oder verschieden sind,
- R⁷ und R⁸: gleich oder verschieden sind und Wasserstoff, Methyl oder Ethyl bedeuten,
oder für geradkettiges oder verzweigtes Alkoxy mit bis zu 3 Kohlenstoffatomen steht, das gegebenenfalls durch Difluormethyl, Difluormethoxy, Trifluormethoxy oder Trifluormethyl substituiert ist,
oder für einen Rest der Formel 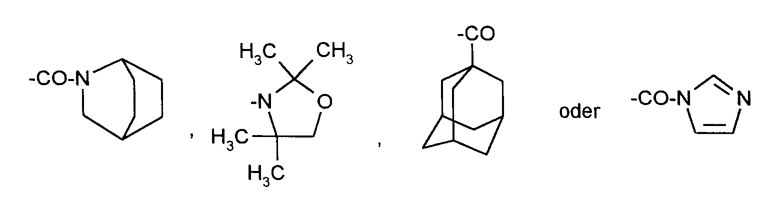
steht,
und deren Salze.

Außerdem wurde ein Verfahren zur Herstellung der erfindungsgemäßen Verbindungen der allgemeinen Formel (I) gefunden, dadurch gekennzeichnet, daß man zunächst
Carbonsäuren der allgemeinen Formel (II)

R¹-CO₂-H (II)

in welcher
- R¹: die oben angegebene Bedeutung hat

durch Umsetzung mit der Verbindung der (III), 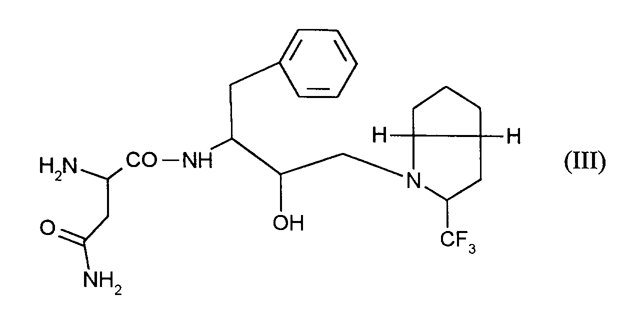
in die Verbindungen der allgemeinen Formel (IV) 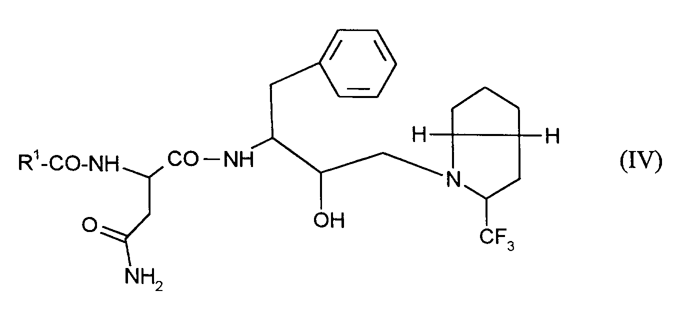
in welcher
- R¹: die oben angegebene Bedeutung hat,
gegebenenfalls unter vorgeschalteter Aktivierung der Carbonsäure, in inerten Lösemitteln und in Anwesenheit einer Base und/oder eines Hilfsmittels, überführt, und in einem zweiten Schritt eine Acylierung, in inerten Lösemitteln, gegebenenfalls in Anwesenheit einen Base und/oder eines Hilfsmittels, durchführt.

Das erfindungsgemäße Verfahren kann durch folgendes Formelschema beispielhaft erläutert werden: 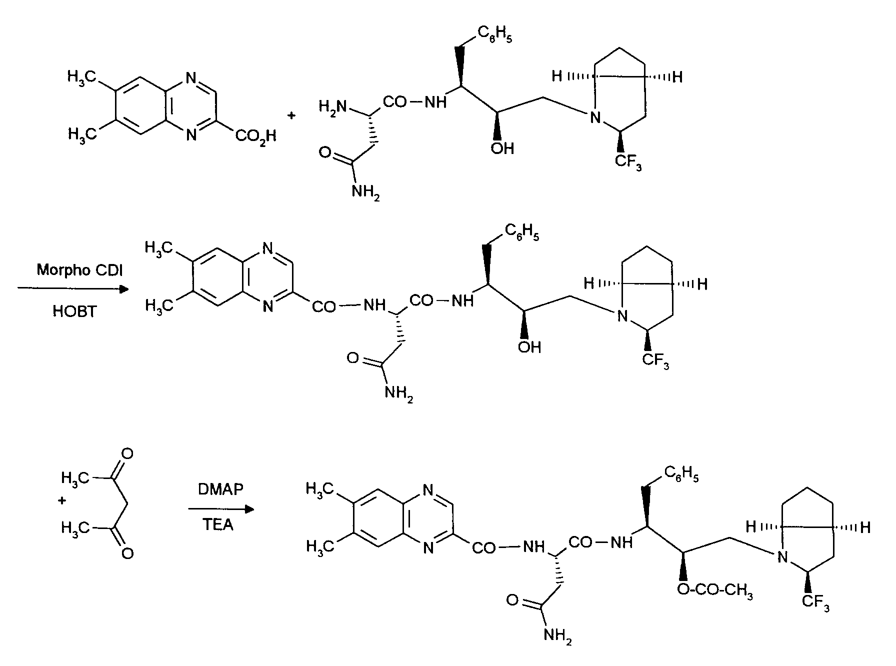
Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dimethylformamid oder Tetrahydrofuran.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine, Dimethylaminopyridin oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 Mol bis 10 Mol, bevorzugt von 1 Mol bis 3 Mol, bezogen auf 1 Mol der Verbindungen der allgemeinen Formeln (II), eingesetzt.

Als Hilfsmittel eingen sich bevorzugt Kondensationsmittel, die auch Basen sein können, insbesondere wenn die Carboxylgruppe als Anhydrid aktiviert vorliegt. Bevorzugt werden hier die üblichen Kondensationsmittel wie Carbodiimide, z.B. N,N-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'-(2-morpholinoethyl)-carbodiimid-metho-p-toluolsulfonat (CMCT bzw. Morpho CDI), oder Carbonylverbindungen wie Carbonyldiimidazol, oder 1,2-Oxazoliumverbindungen wie 2-Ethyl-5-phenyl-1,2-oxazolium-3-sulfat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosponsäureanhydrid, oder Isobutylchloroformat, oder Benzotriazolyloxy-tri(dimethylamino)phosphonium-hexafluorophosphat, 1-Hydroxybenzotriazol oder Dimethylaminopyridin.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Umsetzung erfolgt im allgemeinen in einem Temperaturbereich von -20°C bis +40°C, bevorzugt von 0°C bis Raumtemperatur.

Die Acylierung erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis +50°C, bevorzugt von -10°C bis Raumtemperatur.

Als Basen und/oder Hilfsstoffe für die Acylierung eignen sich im allgemeinen die oben aufgeführten organischen Amine und Pyridine. Bevorzugt sind Triethylamin und Dimethylaminopyridin.

Die Base wird in einer Menge von 1 Mol bis 10 Mol, bevorzugt von 1 Mol bis 3 Mol, bezogen auf 1 Mol des jeweiligen Alkohols, eingesetzt.

Die Carbonsäuren der allgemeinen Formel (II) sind im Fall der Chinoline, Phenyle, Pyridyle und Biphenyle an sich bekannt. Die Chinoxalin-Carbonsäuren sind teilweise bekannt oder neu und können dann beispielsweise durch Umsetzung der entsprechenden Phenylendiamine mit dem Oson der D-Fructose - in situ hergestellt durch Umsetzung des Phenylhydrazons mit NaNO₂ und Salzsäure - und anschließender Oxidation mit H₂O₂-Lösung hergestellt werden [vgl. hierzu G. Henseke, Chem. Ber. 91, 1958, 1605-11 und DE 24 10 852].

Die Verbindung der allgemeinen Formel (III) ist neu und kann beispielsweise hergestellt werden, indem man

Verbindungen der allgemeinen Formel (V) 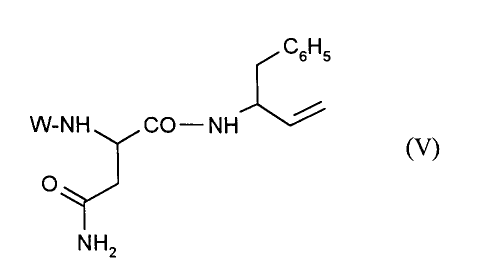
in welcher
- W: für eine Aminoschutzgruppe, vorzugsweise tert.Butoxycarbonyl, steht,
zunächst mit einer Epoxidierungsreaktion, gegebenenfalls mit Hilfe einer Base oder einer Phasentransferkatalyse, in die Verbindungen der allgemeinen Formel (VI), 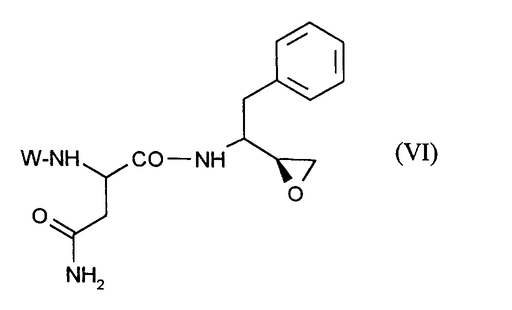
in welcher
- W: die oben angegebene Bedeutung hat,
überführt und anschließend mit 3-Trifluormethyl-2-azabicyclo[3.3.0]octan der Formel (VII) 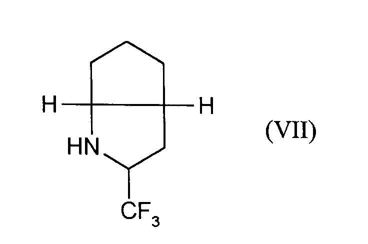
in Lösemitteln umsetzt und die Schutzgruppe nach üblichen Methoden abspaltet.

Als Lösemittel eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid oder n-Propanol.

Als Reagentien für die Epoxidierung eignen sich die literaturbekannten Verbindungen wie beispielsweise m-Chlorbenzoesäure, Magnesiummonoperoxyphthalat, Dimethyldioxiran oder Methyl(trifluormethyl)dioxiran. Bevorzugt sind m-Chlorperbenzoesäure und Magnesiummonoperoxyphthalat [vgl. P. Brongham et al., Synthesis (1987), 1015; W. Adam et al., J. Org. Chem. 52, 2800 (1987) und R. Curci et al., J. Org. Chem. 53, 3890 (1988)].

Wird die Epoxidierung mit Hilfe einer Phasentransfer-Katalyse durchgeführt, so werden als Hilfsstoffe beispielsweise organische Ammoniumchloride- oder -bromide wie beispielsweise Benzyltriethylammoniumchlorid oder -bromid, Methyltrioctylammoniumchlorid, Tetrabutylammoniumbromid, Tricaprylmethylammoniumchlorid (Aliquat 336) eingesetzt. Bevorzugt sind Benzyltriethylammoniumchlorid und -bromid.

Die Epoxidierung wird in einem Temperaturbereich von -10°C bis +90°C, bevorzugt von 0°C bis +60°C, durchgeführt.

Die Reaktionen können sowohl bei Normaldruck als auch bei erhöhtem oder erniedrigtem Druck (beispielsweise 0,5 bis 5 bar), bevorzugt bei Normaldruck, durchgeführt werden.

Die Verbindungen der allgemeinen Formel (IV) sind bekannt und können beispielsweise wie oben beschrieben, hergestellt werden.

Die Verbindungen der allgemeinen Formeln (V) und (VI) sind größtenteils bekannt [vgl. EP 528 242].

Die Verbindung der allgemeinen Formel (VII) ist neu und kann beispielsweise durch Fluorierung von 2-Azabicyclo[3.3.0]octan-3-carbonsäure in der jeweiligen Konfiguration mit HF und SF₄ hergestellt werden.

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen IC₅₀Werte beziehen sich auf die Substanzkonzentration, die unter den oben aufgeführten Testbedingungen eine 50%ige Hemmung der Protease-Aktivität bewirkt.

### Enzym assay, HIV-1

**Tabelle I**

| **Beispiel-Nr.** | **HIV-1-Protease-Inhibition** | |
|---|---|---|
| | **IC**_{**50**} **[Mol/l]** | **IC**_{**95**} **[Mol/l]** |
| 1 | 3,2 x 10⁻⁴ | n.t. |
| 2 | 1,1 x 10⁻⁶ | 3,3 x 10⁻⁵ |
| 3 | 1,5 x 10⁻⁹ | 2,5 x 10⁻⁸ |

Außerdem zeigten die erfindungsgemäßen Verbindungen Wirkung in Lentivirus infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, währen die unbehandelte Zellkontrolle keine Syncytien aufwies.

Die IC₅₀-Werte wurden als die Konzentration der behandelten und infizierten Zellen ermittelt, bei der 50% (ca. 10 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

### Zellkulturassay, PBL

**Tabelle II**

| **Beispiel-Nr.** | **PBL IC**_{**50**} **[Mol/l]** |
|---|---|
| 1 | 1,08 x 10⁻⁷ |
| 2 | 6,12 x 10⁻⁷ |
| 3 | 1,2 x 10⁻⁷ |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen, verursacht durch das Katzenleukämievirus
g) Infektionen ,verursacht durch das Virus der Katzen-Immundefizienz (FIV)
h) Infektionen, verursacht durch das Virus der Affen-Immundefizienz (SIV)
Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Die erfindungsgemäßen Verbindungen sind Enzyminhibitoren und können als solche für alle Zwecke eingesetzt werden für die Enzyminhibitoren brauchbar sind. Beispielhaft ist hier zu nennen der Einsatz als Label für Affinitätschromatographie, die Verwendung als Hilfsmittel für die Aufklärung von Enzymstrukturen und Reaktionsmechanismen sowie der Einsatz als Reagenz für Diagnostika.

### Ausgangsverbindungen

### Beispiel I

### 6,7-Dimethyl-2-[D-arabo-tetrahydroxy-butyl]chinoxalin

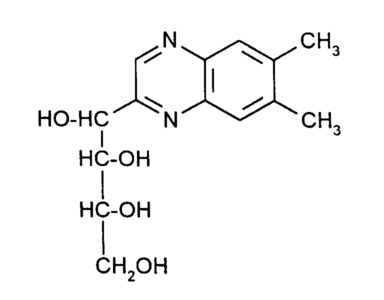

2,0 g (5,58 Mmol) D-Fructose-phenylosazon (hergestellt nach C.L. Butler, J. Am. Chem. Soc. 51, 1929, 3163) werden in 12 ml Wasser, 12 ml Ethanol und 1,4 ml konz. Salzsäure suspendiert, auf 45°C erwärmt und tropfenweise mit einer Lösung von 0,8g (11,2 Mmol) NaNO₂ in 2,5 ml Wasser versetzt. Man erhält eine dunkelrote, klare Lösung, die bei Raumtemperatur mit Natriumacetat abgepuffert wird. Man rührt alles mit 15 ml Essigester aus, trennt die dunkelrote organische Phase ab, engt die gelbe, wäßrige Phase etwas ein und versetzt mit 0,5 g (3,67 Mmol) 4,5-Dimethylphenylendiamin. Man erhitzt das Gemisch 20 Minuten auf dem siedenden Wasserbad und filtert dann den gelben Niederschlag ab. Anschließend rührt man in etwas Methanol aus, filtriert erneut und trocknet.
Ausbeute: 0,3 g (29,4% d.Th.) gelbe Kristalle
Fp.: 190°C (Zers.)
DC: R_{f} (Substanz ist sehr schwer löslich) = 0,4 (Dichlormethan : Methanol = 9:1)

### Beispiel II

### 6,7-Dimethylchinoxalin-2-carbonsäure

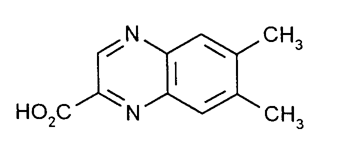

0,3 g (1,08 Mmol) der Verbindung aus Beispiel I werden in 10 ml 10%iger H₂O₂-Lösung suspendiert. Unter Rühren versetzt man mit 0,6 g (15 Mmol) NaOH (fest). Dabei beobachtet man ein Aufschäumen. Nach ca. einer Stunde Rühren erhält man eine klare Lösung. Beim Ansäuern mit konz. Salzsäure fällt ein farbloser Niederschlag aus. Er wird filtriert, mit Wasser gewaschen und getrocknet.
Ausbeute: 160 mg (73,3% d.Th.) farblose Kristalle
Fp.: 203°C (Zers.)
DC: R_{f} = 0,5 (Dichlormethan/Eisessig/Methanol = 90/10/2)

### Beispiel III

### (S,S,S)-3-Trifluormethyl-2-azabicyclo[3.3.0]octan

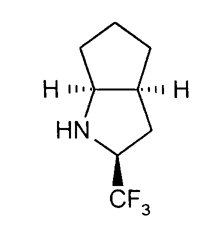

55 g (0,355 Mmol) (S,S,S)-2-Azabicyclo[3.3.0]octan-3-carbonsäure werden mit HF und SF₄ fluoriert. Die dunkelbraune, alkalische Suspension (ca. 200 ml) wird filtriert, i.V. auf ca. 50 ml eingeengt und dreimal mit 50 ml Dichlormethan extrahiert. Die organische Phase wird getrocknet, i.v. auf ein kleines Volumen eingeengt (dunkelbraunes Öl) und säulenchromatographisch getrennt (Kieselgel 60, Dichlormethan).
Ausbeute: 28,8 g (45,3% d.Th.) leicht bräunliches Öl
Fp.: um 0°C, GC: 99%
R_{f} = 0,4 (Dichlormethan)

### Beispiel IV

### (2R,3S)-3-(N-Benzyloxycarbonyl)amino-1-{(S,S,S)-3-trilfüormethyl-2-azabicyclo-[3.3.0]octan-2-yl}-2-hydroxy-4-phenylbutan

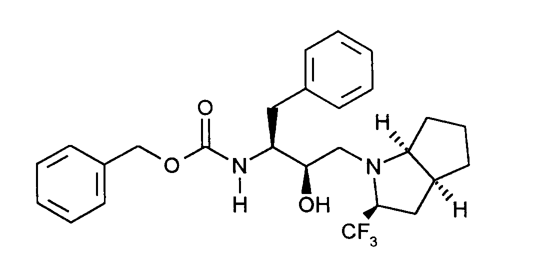

0,3 g (1 Mmol) (2R)-[1-(N-Benzyloxycarbonyl)amino-2-phenyl-(1S)-ethyl]oxiran und 0,15 g (0,84 Mmol) der Verbindung aus Beispiel III werden in 2 ml 2-Propanol gelöst und 12 h in einem geschlossenen Gefäß in einem 130°C heißem Ölbad gerührt. Nach dem Erkalten engt man den Inhalt auf ein kleines Volumen ein und trennt ihn säulenchromatographisch (Kieselgel 60, Toluol/Essigester 100/5).
Ausbeute: 72 mg (18% d.Th.) farbloser Schaum
R_{f} = 0,2 (Toluol / Essigester = 100/5)

### Beispiel V

### (2R,3S)-3-Amino-1-{(S,S,S)-3-trifluormethyl-2-azabicyclo[3.3.0}octan-2-yl}-2-hydroxy-4-phenylbutan

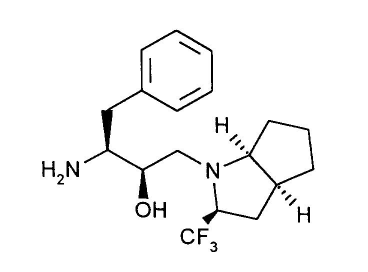

112 mg (0,235 Mmol) der Verbindung aus Beispiel IV werden in 15 ml Methanol/THF (1:1) gelöst, mit etwas Pd/C (10%ig) versetzt und zwei Stunden bei Raumtemperatur hydriert. Nach Abfiltrieren des Katalysators und Abdampfen des Lösemittels erhält man ein leicht gelbes Öl.
Ausbeute: 80 mg (∼quantitativ)
R_{f} =0,6 (Dichlormethan / Methanol =100/5)

### Beispiel VI

### (2R,3S)-3-[N-Benzyloxycarbonyl-L-asparaginyl)amino]-1-{(S,S,S)-3-trifluormethyl-2-azabicylco[3.3.0]octan-2-yl}-2-hydroxy-4-phenyl-butan

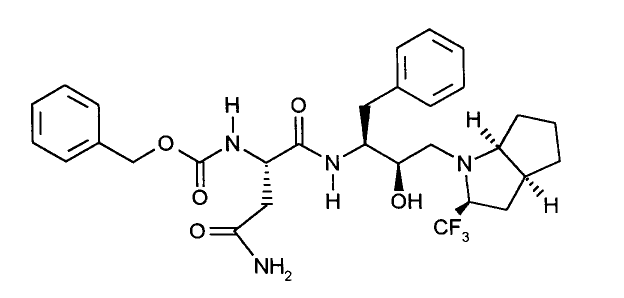

0,074 g (0,28 Mmol) Z-L-Asn-OH und 0,038 g (0,28 Mmol) HOBT (Hydroxybenzotriazol) werden unter Argonatmosphäre in 5 ml DMF gelöst, im Eisbad auf 0°C abgekühlt und unter Rühren mit 0,119 g (0,28 Mmol) MorphoCDI versetzt. Man läßt 3 Stunden bei 0°C rühren und versetzt dann mit 0,08 g (0,234 Mmol) der Verbindung aus Beispiel V, gelöst in 3 ml DMF. Man läßt die Temperatur auf RT ansteigen und rührt weiter über Nacht. Nach Zusatz von Wasser fällt ein farbloser Niederschlag an. Er wird filtriert, mit Wasser gewaschen und getrocknet.
Ausbeute: 87 mg (63,0% d.Th.) farblose Kristalle
Fp.: 162°C
HPLC: 97,53%ig

### Beispiel VII

### (2R,3S)-3-(L-Asparaginyl)amino-1-7(S,S,S)-3-trifluormethyl-2-azabicyclo[3.3.0]-octan-2-yl}-2-hydroxy-4-phenylbutan

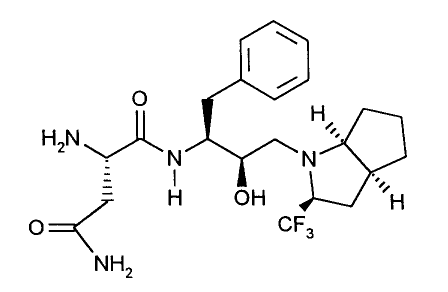

0,008 g (0,135 Mmol) der Verbindung aus Beispiel VI werden in 10ml Methanol/THF (1:1) gelöst, mit etwas Pd/C (10%ig) versetzt und bei RT 2 Stunden hydriert. Nach Abfiltrieren des Katalysators und Abdampfen des Lösemittels i.V. erhält man ein farbloses Öl.
Ausbeute: 58 mg (95% d.Th.)
R_{f} = 0,2 (Dichlormethan/Methanol = 100/5) (Ansprühen mit Ninhydrin)

### Beispiel VIII

### (2R,3S)-3-(Chinoxalin-2-yl-L-asparaginyl)amino-1-({(S,S,S)-3-trifluormethyl-2-azabicyclo[3.3.0]octan-2-yl}-2-hydroxy-4-phenylbutan

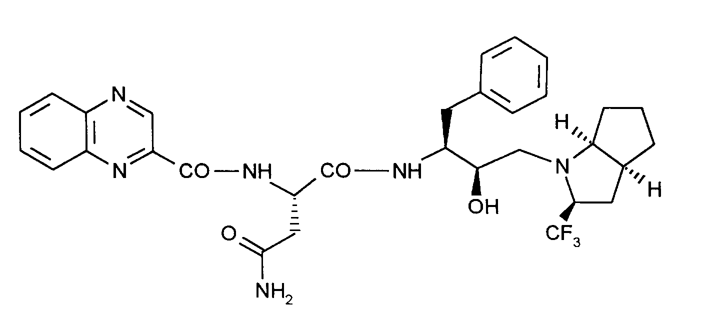

0,095 g (6,7 Mmol) HOBT und 0,071 g (0,7 Mmol) Triethylamin werden unter Argonatmosphäre in 2 ml DMF gelöst, auf -40°C abgekühlt und unter Rühren tropfenweise mit 0,14 g (0,7 Mmol) Chinoxalin-2-carbonsäurechlorid in 3 ml DMF versetzt. Man läßt 2 Stunden im Eisbad reagieren und versetzt dann mit 0,3 g (0,657 Mmol) der Verbindung aus Beispiel VII. Man läßt das Gemisch über Nacht rühren, wobei die Temperatur auf Raumtemperatur ansteigt. Anschließend fällt man das gewünschte Produkt mit Wasser und reinigt es säulenchromatographisch (Kieselgel 60, Laufmittel: Dichlormethan/Methanol = 100/5).
Ausbeute: 130 mg (≙ 32,3% d.Th.) farbloser Schaum
R_{f} =0,5 (Dichlormethan / Methanol =100/5)

### Beispiel IX

### (2R,3S)-3-(Chinolin-2-yl-L-asparaginyl)-amino-1-[(S,S,S)-3-trifluormethyl-2-azabicylco[3.3.0]octan-2-yl}-2-hydroxy-4-phenyl-butan

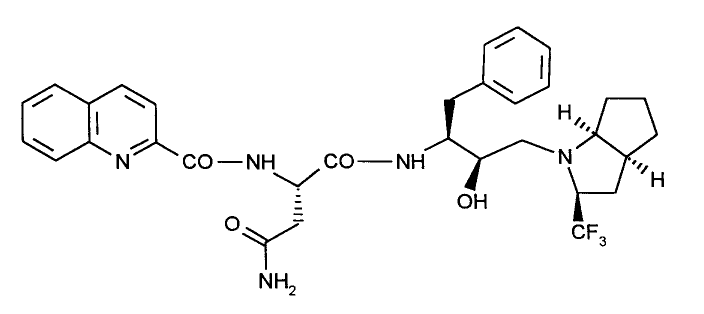

0,014 g (0,08 Mol) Chinolin-2-carbonsäure und 0,011 g (0,08 Mol) HOBT werden unter Argon in 5 ml DMF gelöst, im Eisbad auf 0°C abgekühlt und mit 0,034 g (0,08 Mol) Morpho-CDI versetzt. Nach 3stündigem Rühren bei 0°C gibt man 0,03 g (0,0657 Mol) der Verbindung aus Beispiel VII, gelöst in 1 ml DMF, hinzu und läßt über Nacht rühren, wobei die Temperatur auf Raumtemperatur ansteigt. Anschließend fällt man das gewünschte Produkt durch Wasserzusatz aus, filtriert, trocknet und reinigt es durch Ausfällen mit Methanol und Diisopropylether.
Ausbeute: 270 mg (67,2% d.Th.) farbloses Glas
R_{f} = 0,5 (Dichlormethan / Methanol = 100/5)
In Analogie zu den obigen Beispielen werden die folgenden neuen Pseudopeptide mit trifluormethyl-substituiertem 2-Azabicyclooctan hergestellt, Tabelle I:

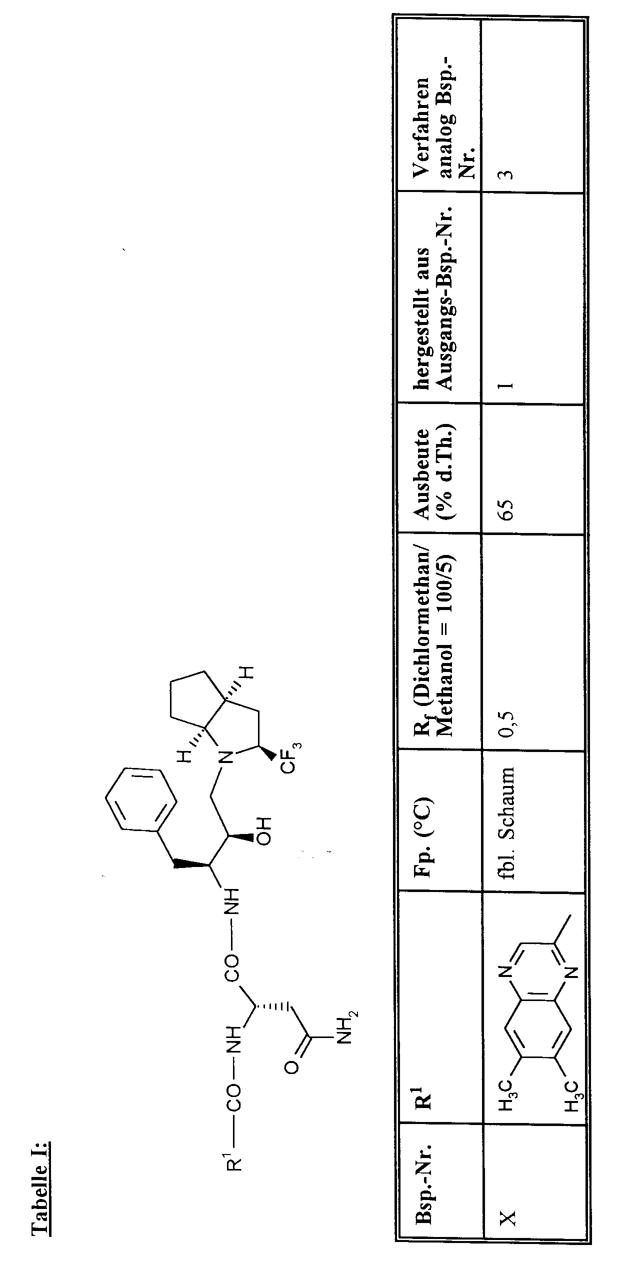

### Herstellungsbeispiele

### Beispiel 1

### (2R,3S)-3-(Chinolin-2-yl-L-asparaginyl)-amino-1-[(S,S,S)-3-trifluormethyl-2-azabicylco[3.3.0]octan-2-yl}-2-(3-ethoxycarbonyl)propionyl-4-phenylbutan

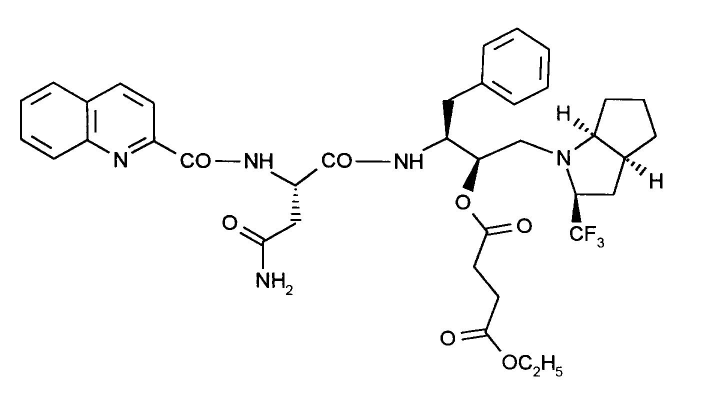

0,6 g (0,981 Mmol) der Verbindung aus Beispiel IX werden mit 0,4 g (3,93 Mmol ≙ 0,54 ml) Triethylamin (d = 0,73), 0,32 g (1,96 Mmol ≙ 0,278 ml) Bernsteinsäureethylesterchlorid (d = 1,15) und 0,6 g (4,92 Mmol) Dimethylaminopyridin (DMAP) in 15 ml getrocknetem THF unter Argon bei Raumtemperatur über Nacht gerührt. Der Ansatz wird mit Wasser versetzt, der Niederschlag abgetrennt, mit Wasser gewaschen, in Toluol gelöst, getrocknet und eingeengt.
Ausbeute: 0,58 g (≙ 80% d.Th.) farblose Kristalle
Fp.: 138°C
R_{f} ∼ 0,6 (Dichlormethan/Methanol = 100/5)
HPLC: 92,5%
rekristallisiert aus Dichlormethan / Diisopropylether => HPLC: 94,2%
In Analogie zur Vorschrift des Beispiels 1 werden die in der Tabelle 1 aufgeführten Verbindungen hergestellt:

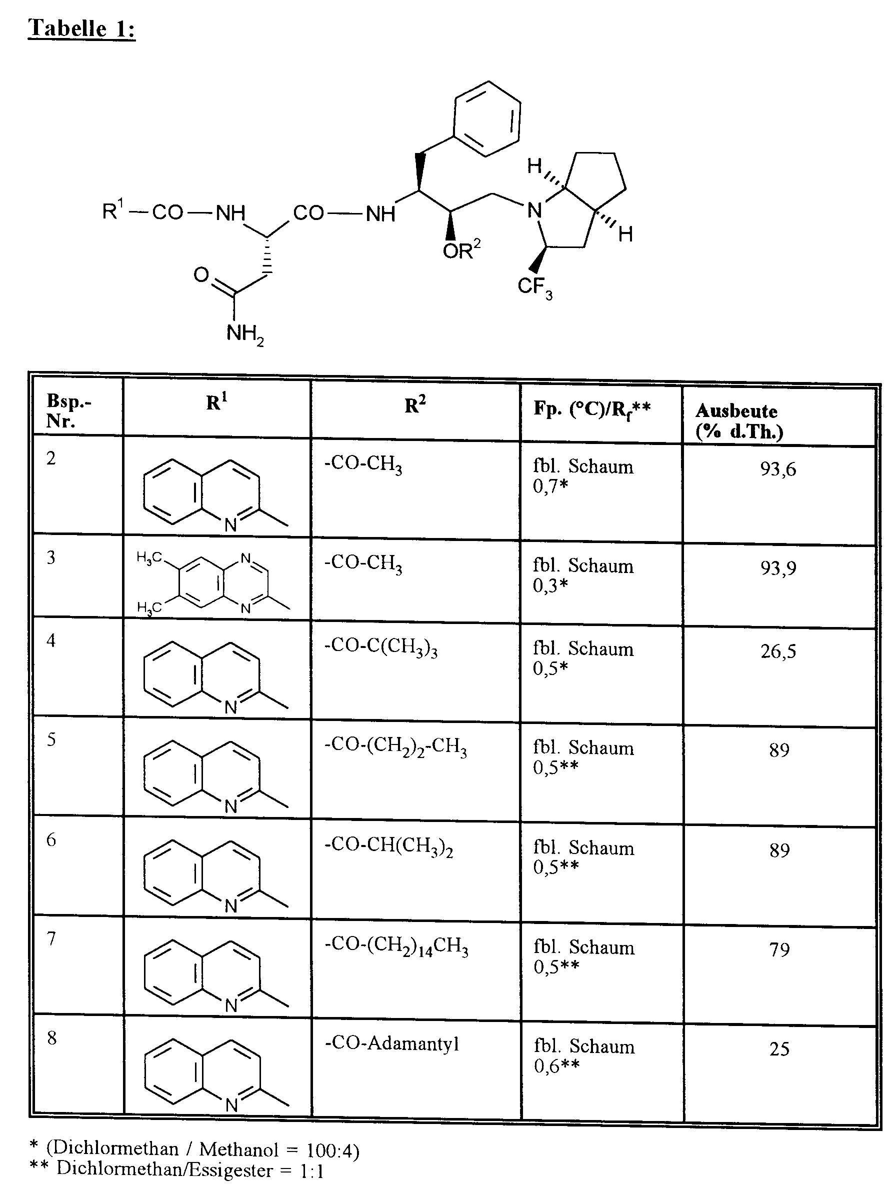

## Patentansprüche

1. Pseudopeptide mit trifluoromethyl-substituiertem 2-Azabicyclooctan der allgemeinen Formel (I) 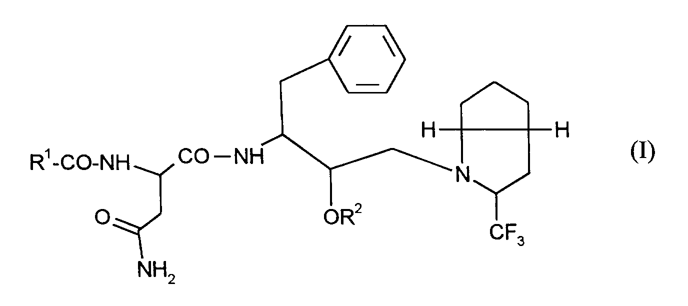 in welcher
R¹ für einen Rest der Formel 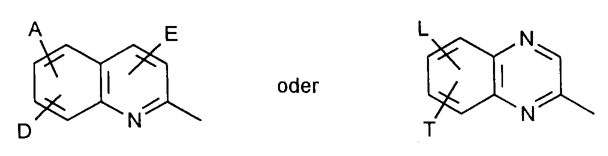 steht,
worin
A, D, E, L und T gleich oder verschieden sind und Wasserstoff, Halogen, geradkettiges oder verzweigtes Alkyl mit bis zu 4 Kohlenstoffatomen, Morpholinyl, Phenylthio oder Cycloalkyl mit 3 bis 6 Kohlenstoffatomen bedeuten,
R² für geradkettiges oder verzweigtes Acyl mit bis zu 20 Kohlenstoffatomen steht, das gegebenenfalls durch Carboxy, durch geradkettiges oder verzweigtes Alkoxycarbonyl mit bis zu 6 Kohlenstoffatomen oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 6 Kohlenstoffatomen bedeuten,
für einen Rest der Formel -CO-NR⁵R⁶ oder P(O)(OR⁷)(OR⁸) steht,
worin
R⁵ und R⁶ die oben angegebene Bedeutung von R³ und R⁴ haben und mit dieser gleich oder verschieden sind,
R⁷ und R⁸ gleich oder verschieden sind und Wasserstoff oder geradkettiges oder verzweigtes Alkyl mit bis zu 4 Kohlenstoffatomen bedeuten,
oder für geradkettiges oder verzweigtes Alkoxy mit bis zu 6 Kohlenstoffatomen steht, das gegebenenfalls durch Difluormethyl, Difluormethoxy, Trifluormethoxy oder Trifluormethyl substituiert ist,
oder für einen Rest der Formel 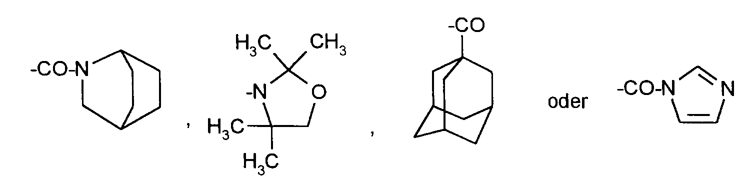 steht,
und deren Salze.

2. Verbindungen der allgemeinen Formel (I), gemäß Anspruch 1
in welcher
R¹ für einen Rest der Formel 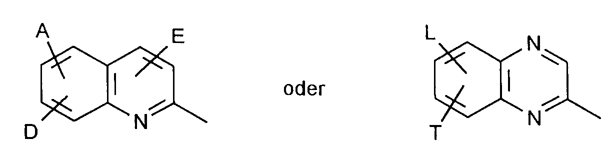 steht,
worin
A, D, E, L und T gleich oder verschieden sind und sind und Wasserstoff, Fluor, Chlor, Brom, geradkettiges oder verzweigtes Alkyl mit bis zu 3 Kohlenstoffatomen, Morpholinyl, Phenylthio, Cyclopentyl oder Cylcohexyl bedeuten,
R² für geradkettiges oder verzweigtes Acyl mit bis zu 17 Kohlenstoffatomen steht, das gegebenenfalls durch Carboxy, geradkettiges oder verzweigtes Alkoxycarbonyl mit bis zu 4 Kohlenstoffatomen oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 4 Kohlenstoffatomen bedeuten,
für einen Rest der Formel -CO-NR⁵R⁶ oder P(O)(OR⁷)(OR⁸) steht,
worin
R⁵ und R⁶ die oben angegebene Bedeutung von R³ und R⁴ haben und mit dieser gleich oder verschieden sind,
R⁷ und R⁸ gleich oder verschieden sind und Wasserstoff oder geradkettiges oder verzweigtes Alkyl mit bis zu 3 Kohlenstoffatomen bedeuten,
oder für geradkettiges oder verzweigtes Alkoxy mit bis zu 4 Kohlenstoffatomen steht, das gegebenenfalls durch Difluormethyl, Difluormethoxy, Trifluormethoxy oder Trifluormethyl substituiert ist,
oder für einen Rest der Formel 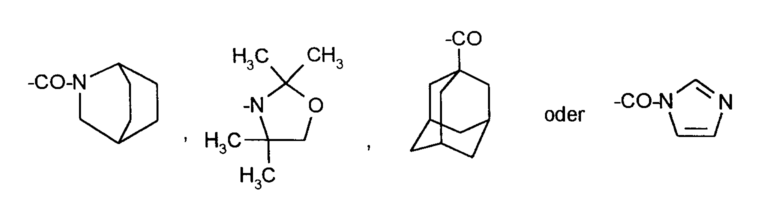 steht,
und deren Salze.

3. Verbindungen der allgemeinen Formel (I), gemäß Anspruch 1
in welcher
R¹ für einen Rest der Formel 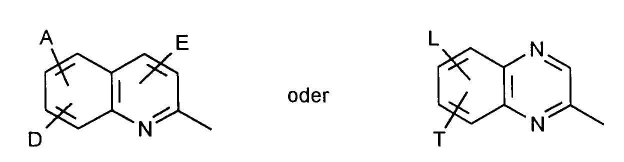 steht,
worin
A, D, E, L und T gleich oder verschieden sind und sind und Wasserstoff, Fluor, Chlor, Brom, Methyl, Phenylthio, Cyclopentyl oder Cyclohexyl bedeuten,
R² für geradkettiges oder verzweigtes Acyl mit bis zu 15 Kohlenstoffatomen steht, das gegebenenfalls durch Carboxy, geradkettiges oder verzweigtes Alkoxycarbonyl mit bis zu 6 Kohlenstoffatomen oder durch eine Gruppe der Formel -NR³R⁴ substituiert ist,
worin
R³ und R⁴ gleich oder verschieden sind und Wasserstoff, Phenyl oder geradkettiges oder verzweigtes Alkyl mit bis zu 3 Kohlenstoffatomen bedeuten,
für einen Rest der Formel -CO-NR⁵R⁶ oder P(O)(OR⁷)(OR⁸) steht,
worin
R⁵ und R⁶ die oben angegebene Bedeutung von R³ und R⁴ haben und mit dieser gleich oder verschieden sind,
R⁷ und R⁸ gleich oder verschieden sind und Wasserstoff, Methyl oder Ethyl bedeuten,
oder für geradkettiges oder verzweigtes Alkoxy mit bis zu 3 Kohlenstoffatomen steht, das gegebenenfalls durch Difluormethyl, Difluormethoxy, Trifluormethoxy oder Trifluormethyl substituiert ist,
oder für einen Rest der Formel 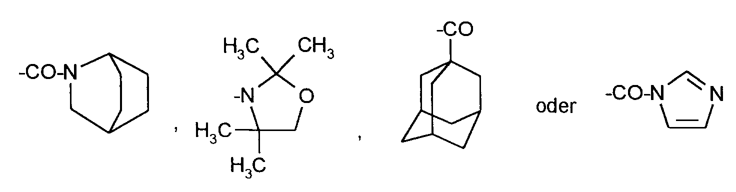 steht,
und deren Salze.

4. Verfahren zur Herstellung der Verbindungen der allgemeinen Formel (I) gemäß Anspruch 1, dadurch gekennzeichnet, daß man
zunächst
Carbonsäuren der allgemeinen Formel (II)
R¹-CO₂-H (II)
in welcher
R¹ die im Anspruch 1 angegebene Bedeutung hat
durch Umsetzung mit der Verbindung der (III), 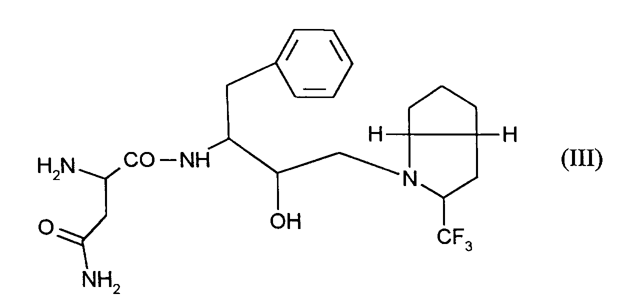 in die Verbindungen der allgemeinen Formel (IV) 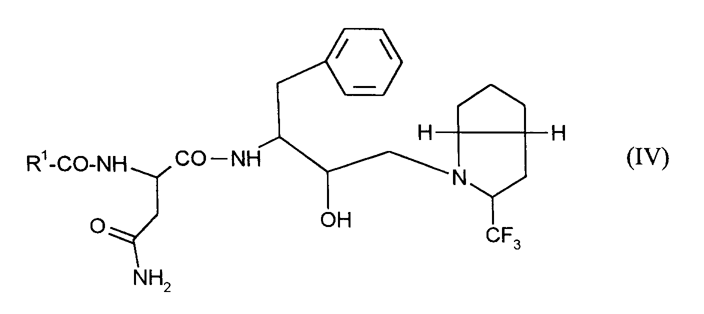 in welcher
R¹ die im Anspruch 1 angegebene Bedeutung hat,
gegebenenfalls unter vorgeschalteter Aktivierung der Carbonsäure, in inerten Lösemitteln und in Anwesenheit einer Base und/oder eines Hilfsmittels, überführt, und in einem zweiten Schritt eine Acylierung, in inerten Lösemitteln, gegebenenfalls in Anwesenheit einen Base und/oder eines Hilfsmittels, durchführt.

5. Arzneimittel enthaltend eine oder mehrere Verbindungen gemäß Anspruch 1.
